(19) Europäisches Patentamt
European Patent Office
Office européen des brevets

(11) Veröffentlichungsnummer: **0 278 020 B1**

# (12) EUROPÄISCHE PATENTSCHRIFT

(45) Veröffentlichungstag der Patentschrift: **24.05.89**

(51) Int. Cl.⁴: **C07D 321/06**, C07D 407/04, A61K 7/46, C11B 9/00

(21) Anmeldenummer: **87101732.3**

(22) Anmeldetag: **09.02.87**

## (54) 2-Substituierte 4,4,7,7-Tetramethyl-1,3-dioxacycloheptane,

(43) Veröffentlichungstag der Anmeldung: **17.08.88 Patentblatt 88/33**

(45) Bekanntmachung des Hinweises auf die Patenterteilung: **24.05.89 Patentblatt 89/21**

(84) Benannte Vertragsstaaten: **CH DE ES FR GB IT LI NL**

(56) Entgegenhaltungen:
**CH-A- 590 058**
**US-A- 3 122 562**
**US-A- 3 966 768**

**CHEMICAL ABSTRACTS, Band 86, Nr. 13, 28. März 1977, Seite 410, Spalten 1-2, Zusammenfassungsnr. 95863w, Columbus, Ohio, US; G.M. FILIPPOVA et al.: "Synthesis of cyclic acetals and a perfumery evaluation of their odor", & MASLO-ZHIR. PROM. ST. 1976, (6), 30-32 000**
**CHEMICAL ABSTRACTS, Band 86, Nr. 15, 11. April 1977, Seite 293, Spalte 2, Zusammenfassungsnr. 111086c, Columbus, Ohio, US; S.A. VOITKEVICH et al.: "Gas-chromatographic determination of the saturation vapor pressure of perfumes. III. Cyclic acetals of aldehydes of the aliphatic series and phenyl-substituted aldehydes and alcohols", & MASLO-ZHIR. PROM-ST. 1977, (1), 35-37**

(73) Patentinhaber: **BASF Aktiengesellschaft, Carl-Bosch-Strasse 38, D-6700 Ludwigshafen(DE)**

(72) Erfinder: **Gramlich,Walter,Dr., Auf der Hoehe 11, D-8603 Edingen-Neckarhausen(DE)**
Erfinder: **Siegel,Hardo,Dr., Hans-Purrman-Allee 25, D-6720 Speyer(DE)**

**EP 0 278 020 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft 2-substituierte 4,4,7,7-Tetramethyl-1,3-dioxacycloheptane der allgemeinen Formel I

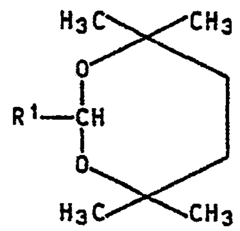

(I)

in der $R^1$ einen geradkettigen oder verzweigten Alkylrest mit 1 bis 10 C-Atomen, der zusätzlich Sauerstoff in Form einer Ethergruppierung enthalten kann oder einen 5- bis 8-gliedrigen Cycloalkylring mit bis zu 10 C-Atomen, der zusätzlich Sauerstoff in Form einer Ethergruppierung enthalten kann, bedeutet, deren Herstellung und Verwendung als Riechstoffe.

Wegen der im allgemeinen von vielen unwägbaren Faktoren abhängigen unsicheren Verfügbarkeit vieler natürlicher Riechstoffkomponenten sowie der notwendigen Anpassung an wechselnde modische Geschmacksrichtungen hat die Riechstoffindustrie ständig Bedarf an neuen Riechstoffen, die entweder allein oder in Form von Kompositionen wertvolle Parfüms mit interessanten Duftnoten darstellen. Darüber hinaus ist ein ständig wachsender Trend zur Parfümierung von Produkten des täglichen Bedarfs, wie Kosmetika und technischen Artikeln wie Leimen, Wasch- und Reinigungsmitteln, für Raumsprays und anderem festzustellen.

Da aufgrund wenig bekannter Zusammenhänge zwischen Struktur und olfaktorischen Eigenschaften eine gezielte Synthese von Riechstoffen mit gewünschter Geruchsqualität nicht möglich ist, besteht die Aufgabe, solche Verbindungen aufzufinden, die wertvolle Riechstoffeigenschaften aufweisen.

Es wurde nun gefunden, daß die oben beschriebenen Verbindungen der Formel I hochinteressante Riechstoffe darstellen.

Über strukturverwandte Verbindungen vom Typ substituierter Dioxacycloheptane wurde in der Literatur bisher meist im Zusammenhang mit theoretischen Studien, wie Konformationsuntersuchungen (vgl. T.B. Grindley u.a. in J. Amer. Chem. Soc. 103 (1981) Seiten 936-938) berichtet.

In 2-Stellung monoalkylierte Dioxacycloheptane und deren Verwendung als Riechstoffe für Parfums sind im Chemical Abstracts 86, Zusammenfassungssummen 95863w und 111086c erwähnt.

In 2-Stellung mono- oder dialkylierte 4,7-Dihydro-1,3-dioxepine (1,3-Dioxa-5-cycloheptene) der Formel IV

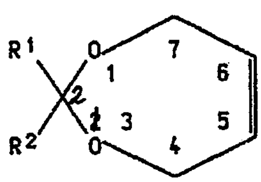

(IV)

($R^1$ und $R^2$ = H oder Alkyl)

sind aus der Literatur (s. M.J. Soulier u.a., Bull. Soc. Chim. Fr. 1975, Seiten 1763-66) als Herbizide und Polymerisations-Katalysatoren sowie als Vorprodukte für Riechstoffe (s. EP 24 473) bekannt. Sie selbst wurden in loc. cit. nicht als Riechstoffe erwähnt.

Aus CH-A 590 058 sind die Verbindungen 4,7-Dihydro-2-(3-pentyl)- 1,3-dioxepin und 4,7-Dihydro-2-isopentyl-2-methyl-1,3-dioxepin sowie deren Verwendung als Aroma- und Riechstoffe bekannt. In dieser Druckschrift wird aber ausdrücklich darauf hingewiesen, daß die entsprechenden gesättigten cyclischen Acetale wie das 2-(3-Pentyl)-1,3- dioxacycloheptan und das 2-Isopentyl-2-methyl-1,3-dioxacycloheptan, keine nützlichen Aromen- oder Riechstoffeigenschaften aufweisen.

Weiterhin sind das 4,7-Dimethyl-4,7-dihydro-1,3-dioxepin und das 2-tert.-Butyl-4,7-dimethyl.-4,7-dihydro-1,3-dioxepin aus einer Arbeit über Konformationsanalysen in J. Org. Chem. Vol. 40, No. 4 (1975), Seiten 450-453 bekannt. Anmerkungen über eventuelle Riechstoffqualitäten finden sich in der Arbeit nicht.

Darüber hinaus sind in 2-Stellung unsubstituiertes 4,4,7,7-Tetramethyl-4,7-dihydro-1,3-dioxepin und 4,7-Dimethyl-4,7-diethyl-4,7-dihydro-1,3-dioxepin aus CA69:P19996a als Komponente für die Herstellung von Co-Polymeren bekannt. Auch in den zuletzt genannten Literaturstellen findet sich keinerlei Hinweis darüber, daß Verbindungen mit der 1,3-Dioxepin-Struktur als Riechstoffe interessant sein könnten.

Es war daher sehr überraschend, daß die erfindungsgemäßen in 2-Stellung substituierten 4,4,7,7-Tetramethyl-1,3-dioxacycloheptane Verbindungen mit hochinteressanten Geruchsnoten darstellen und daher als Riechstoffe verwendet werden können.

Gegenstand der Anmeldung ist daher auch die Verwendung der Verbindungen der allgemeinen Formel I als Riechstoffe sowie Riechstoffkompositionen, gekennzeichnet durch einen Gehalt von 1 bis 50 Gew.% an den in 2-Stellung substituierten 4,4,7,7-Tetramethyl-1,3-dioxacyloheptanen der Formel I.

Gegenstand der Erfindung ist ferner ein Verfahren zur Herstellung der 2-substituierten 4,4,7,7-Tetramethyl-1,3-dioxacycloheptane der allgemeinen Formel I gemäß Anspruch 1, das dadurch gekennzeichnet ist daß man einen Aldehyd der allgemeinen Formel II

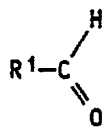

(II)

in der $R^1$ die in Anspruch 1 angegebene Bedeutung hat, in an sich bekannter Weise mit 2,5-Dimethyl-hexan-2,5-diol der Formel III

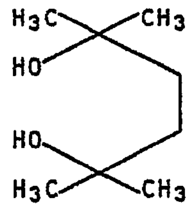

(III)

umsetzt,
sowie ein Verfahren zur Herstellung der 2-substituierten 4,4,7,7-Tetramethyl-1,3-dioxacycloheptane der allgemeinen Formel I gemäß Anspruch 1, das dadurch gekennzeichnet ist, daß man die entsprechenden neuen 2-substituierten 4,4,7,7-Tetramethyl-1,3-dioxa-5-cycloheptene der allgemeinen Formel IV

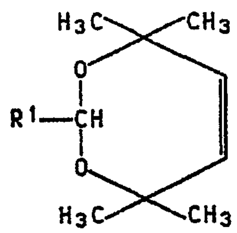

(IV)

in an sich bekannter Weise hydriert.

Die Herstellung von 2-substituierten 4,4,7,7-Tetramethyl-1,3- dioxa-5-cycloheptenen ist in EP-A 0 221 444 (Stand der Technik nach Art. 54(3) EPÜ) beschrieben.

Die Umsetzung der Aldehyde II mit III erfolgt nach prinzipiell literaturbekannten Methoden der Cyclisierung, so daß sich detaillierte Angaben hierüber erübrigen. Bezüglich näherer Einzelheiten verweisen wir auf Houben-Weyl, "Methoden der organischen Chemie", Band VI/3 (1965), Seiten 204 ff. Mit Vorteil arbeitet man in Gegenwart eines Cyclisierungskatalysators, wie p-Toluolsulfonsäure, Phosphorsäure oder eines sauren Ionenaustauschers, und in Gegenwart eines Schleppmittels, wie Toluol oder Cyclohexan, zur Entfernung des bei der Cyclisierung entstehenden Wassers.

Als Aldehyde der Formel II sind beispielsweise Acetaldehyd, Propionaldehyd, 2-Methyl-propanal, n-Butyraldehyd, 2-Methyl-butanal, 3-Methyl-butanal, 2-Ethyl-butanal, n-Valeraldehyd, 2-Methyl-pentanal, 3-Methyl-pentanal, 4-Methyl-pentanal, 2,2-Dimethyl-propanal (Pivalaldehyd), 2,2-Dimethyl-butanal und 2,2-Dimethyl-pentanal geeignet. Aber auch unsubsti tuierte höhere Aldehyde, wie n-Hexanal, n-Heptanal, n-Octanal, n-Nonanal, Decanal sowie deren ein- und/oder mehrfach alkylierte Derivate können eingesetzt werden. Bekannteste Vertreter sind das chemische Großprodukt 2-Ethyl-hexanal sowie 3,5,5-Trimethyl-hexanal.

Als cyclische Aldehyde kommen beispielsweise Cyclopentylcarbaldehyd und Cyclohexancarbaldehyd, als Sauerstoff enthaltende Vertreter beispielsweise Methoxyacetaldehyd und Tetrahydropyran-3-carbaldehyd in Betracht.

2,5-Dimethylhexandiol-2,5 ist ein handelsübliches bedeutendes technisches Zwischenprodukt.

Für die Hydrierung der neuen 1,3-Dioxa-5-cycloheptene der Formel IV kommen alle für die Hydrierung von C-C-Doppelbindungen geeigneten Katalysatoren, wie Palladium auf verschiedenen Trägern (Aktivkohle, Aluminiumoxid, Titandioxid, Tonerde), Raney-Nickel sowie Ruthenium und Platin auf verschiedenen Trägern in Betracht.

Die notwendigen Wasserstoffdrücke hängen vom eingesetzten Katalysator und dessen Gewichtsanteil ab. Die Hydrierung ist problemlos, da keine hydrierbaren funktionellen Gruppen im Molekül anwesend sind. Bezüglich näherer Einzelheiten über solche Hydrierungen verweisen wir auf Houben-Weyl, "Methoden der organischen Chemie", Band IV/1c (1980), Seiten 145 ff. Im Normalfall wird man jedoch die direkte Cyclisierung ausgehend von dem billigen Dimethylhexandiol zur Synthese der neuen Verbindungen anwenden.

Die neuen 4,4,7,7-Tetramethyl-1,3-dioxacycloheptane der Formel I lassen sich gut mit anderen Riechstoffen in verschiedenen Mengenverhältnissen zu neuartigen, interessanten Riechstoffkompositionen mischen.

Außer in der Feinparfümerie können derartige Kompositionen zur Parfümierung von Kosmetika wie Cremes, Lotionen, Aerosolen, Toilettenseifen, technischen Artikeln wie Reinigungs- und Waschmitteln, Weichspüler, Desinfektionsmittel und Textilbehandlungsmittel dienen.

Die neuen Verbindungen zeichnen sich besonders durch ihre Frische-Note aus und können daher bestehende Kompositionen stark aufwerten. Da sie außer der Acetalgruppierung keine weitere funktionelle Gruppe tragen, sind sie im neutralen und alkalischen Medium sehr stabil.

Besonders wertvolle Verbindungen als Riechstoffe für die Feinparfümerie sind:
2-tert.-Butyl-4,4,7,7-tetramethyl-1,3-dioxacycloheptan, 2,4,4,7,7-Pentamethyl-1,3-dioxacycloheptan und 2-Isopropyl-4,4,7,7-tetramethyl-1,3-dioxacycloheptan.

Die folgenden Beispiele sollen den Gegenstand der Erfindung näher erläutern.

<u>Beispiel 1</u>

2-tert.-Butyl-4,4,7,7-tetramethyl-1,3-dioxacycloheptan

Ein Gemisch aus 730 g (5 mol) 2,5-Dimethyl-hexan-2,5-diol, 3 g p-Toluolsulfonsäure, 430 g (5 mol) Pivalaldehyd und 1 l Toluol wurde unter Rückfluß zum Sieden erhitzt, wobei innerhalb von 4 Stunden (h) das anfallende Reaktionswasser (90 ml) entfernt wurde. Anschließend ließ man das Reaktionsgemisch auf Raumtemperatur (RT) abkühlen, rührte mit 10 ml einer 25 %igen wäßrigen Natronlauge aus, wusch einmal mit wenig Wasser neutral und entfernte danach das Toluol bei 50 bis 60°C. Der erhaltene Rückstand wurde unter vermindertem Druck fraktioniert. Man erhielt 909 g (4,25 mol) 2-tert.-Butyl-4,4,7,7-tetramethyl-1,3-dioxacycloheptan (Kp. 54°C/0,2 mbar). Das entspricht einer Ausbeute von 85 % der Theorie.

<u>Beispiel 2</u>

2-Isopropyl-4,4,7,7-tetramethyl-1,3-dioxacycloheptan

In einem 300 ml Autoklaven wurde ein Gemisch aus 60 g 2-Isopropyl-4,4,7,7-tetramethyl-1,3-dioxa-5-cyclohepten, 70 ml Essigsäureethylester und 10 g eines 0,5 % Palladium/Aluminiumoxid-Katalysators bei 150°C und 150 bar Wasserstoffdruck hydriert.

Nach Entfernen des Katalysators und des Essigesters erhielt man durch Destillation 52 g 2-Isopropyl-4,4,7,7-tetramethyl-1,3-dioxacycloheptan, Kp. 77°C/23 mbar.

Die physikalischen Eigenschaften der in den Beispielen 1 und 2 hergestellten Verbindungen sowie analog Beispiel 1 hergestellter anderer olfaktorisch interessanter erfindungsgemäßer Verbindungen der Formel I sind in der folgenden Tabelle zusammengestellt.

**Tabelle**

| Nr. | 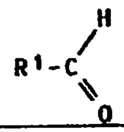 | -4,4,7,7-tetramethyl-1,3-dioxacycloheptane | Kp. [°C/mbar] | $n_D^{25}$ | Geruchsbeschreibung |
|---|---|---|---|---|---|
| 1 | Pivalaldehyd | 2-tert.-Butyl- | 54/0,2 | 1.4338 | sehr intensive und frische Grün-Note (Rose, Maiglöckchen), Civet |
| 2 | Isobutyraldehyd | 2-Isopropyl- | 77/23 | 1.4337 | frische intensive Note, Muskatellersalbei-artig |
| 3 | Acetaldehyd | 2-Methyl- | 68/26 | 1.4310 | starke Campher-Note, kühlender Effekt, intensiv, frisch |
| 4 | Propanal | 2-Ethyl- | 74/22 | 1.4333 | frische krautig-fruchtige Note, Methylchavicolartig |
| 5 | Butanal | 2-Propyl- | 88/24 | 1.4362 | frische fruchtige Note |
| 6 | Pentanal | 2-Butyl- | 50/0,3 | 1.4386 | krautige Grün-Note |
| 7 | 3-Methyl-butanal | 2-(2-Methyl-propyl) | 27/0,2 | 1.4367 | krautige Minze-Note |
| 8 | 2-Methyl-pentanal | 2-(1-Methyl-butyl) | 63/0,2 | 1.4401 | schwache krautige Note |
| 9 | Cyclopentyl-carbaldehyd | 2-Cyclopentyl | 48/0,03 | 1.4595 | krautige Note, Sellerie-artig |
| 10 | Methoxyacetaldehyd | 2-Methoxy-methyl | 98/42 | 1.4382 | frische Minze-Note, grün, blumig |

Anwendungsbeispiel

Die großen Einsatzmöglichkeiten der neuen Verbindungen sollen anhand der Wirkung von 2-tert.-Butyl-4,4,7,7-tetramethyl-1,3-dioxacycloheptan auf eine blumenartige Phantasiekomposition A (vgl. G. Ohloff, H. Rode-Gowal in "Kosmetika, Riechstoffe und Lebensmittelzusatzstoffe", Georg Thieme Verlag, Stuttgart 1978, Seite 61) angedeutet werden.

| Komponenten | Komposition A (Anteil) | Komposition B (Anteil) |
|---|---|---|
| Hydroxycitronellal | 100 | 0 |
| 2-tert.-Butyl-4,4,7,7-tetramethyl-1,3-dioxacycloheptan | 0 | 100 |
| 2-Phenyl-ethanol | 50 | 50 |
| Ylang (synthetisch) | 100 | 100 |
| Indanmoschus | 20 | 20 |
| Coumarin | 80 | 80 |
| Linalool | 200 | 200 |
| Linalylacetat | 150 | 150 |
| Benzylacetat | 50 | 50 |
| Bergamotte (synthetisch) | 50 | 50 |
| Gemisch aus 1-(3,4-Epoxy-4-methylpentyl)4- und 5-formyl-cyclohexen | 100 | 100 |
| | 1000 | 1000 |

Der Ersatz von Hydroxycitronellal in der Komposition A durch 2-tert.-Butyl-1,3-dioxacycloheptan führt zu der Komposition B, die eine erheblich frischere und viel natürlichere Gesamtnote aufweist.

**Patentansprüche**

1. 2-substituierte 4,4,7,7-Tetramethyl-1,3-dioxacycloheptane der allgemeinen Formel I

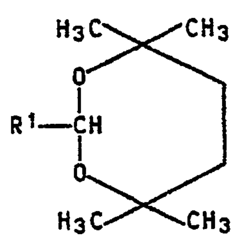

(I)

in der $R^1$ einen geradkettigen oder verzweigten Alkylrest mit 1 bis 10 C-Atomen, der zusätzlich Sauerstoff in Form einer Ethergruppierung enthalten kann oder einen 5- bis 8-gliedrigen Cycloalkylring mit bis zu 10 C-Atomen, der zusätzlich Sauerstoff in Form einer Ethergruppierung enthalten kann, bedeutet.

2. 2,4,4,7,7-Pentamethyl-1,3-dioxacycloheptan.

3. 2-tert.-Butyl-4,4,7,7-tetramethyl-1,3-dioxacycloheptan.

4. 2-Isopropyl-4,4,7,7-tetramethyl-1,3-dioxacycloheptan.

5. Verwendung der 2-substituierten 4,4,7,7-Tetramethyl-1,3-dioxacycloheptane der Formel I gemäß Anspruch 1 als Riechstoffe.

6. Riechstoffkompositionen, gekennzeichnet durch einen Gehalt von 1 bis 50 Gew.% an den 2-substituierten 4,4,7,7-Tetramethyl-1,3-dioxacycloheptanen der allgemeinen Formel I gemäß Anspruch 1.

7. Verfahren zur Herstellung der 2-substituierten 4,4,7,7-Tetramethyl-1,3-dioxacycloheptane der allgemeinen Formel I gemäß Anspruch 1, dadurch gekennzeichnet, daß man einen Aldehyd der allgemeinen Formel II

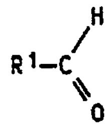

(II)

in der $R^1$ die in Anspruch 1 angegebene Bedeutung hat, in an sich bekannter Weise mit 2,5-Dimethyl-hexan-2,5-diol der Formel III

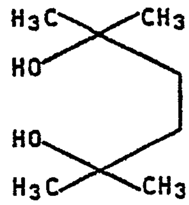

(III)

umsetzt.

8. Verfahren zur Herstellung der 4,4,7,7-Tetramethyl-1,3-dioxacycloheptane der allgemeinen Formel I gemäß Anspruch 1, dadurch gekennzeichnet, daß man die entsprechenden 4,4,7,7-Tetramethyl-1,3-dioxa-5-cycloheptene der allgemeinen Formel IV

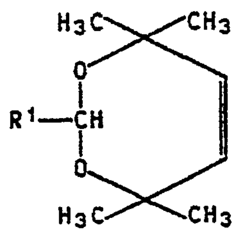

(IV)

in an sich bekannter Weise hydriert.

**Claims**

1. A 2-substituted 4,4,7,7-tetramethyl-1,3-dioxacycloheptane of the general formula I

I

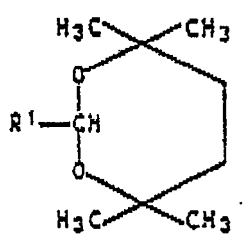

(I)

where $R^1$ is straight-chain or branched alkyl of 1 to 10 carbon atoms which additionally may contain oxygen in the form of an ether group or is 5- to 8-membered cycloalkyl of up to 10 carbon atoms which may additionally contain oxygen in the form of an ether group.

2. 2,4,4,7,7-Pentamethyl-1,3-dioxacycloheptane.

3. 2-tert.-Butyl-4,4,7,7-tetramethyl-1,3-dioxacycloheptane.

4. 2-Isopropyl-4,4,7,7-tetramethyl-1,3-dioxacycloheptane.

5. Use of a 2-substituted 4,4,7,7-tetramethyl-1,3-dioxacycloheptane of the formula I as claimed in claim 1 as a scent.

6. A scent composition which contains from 1 to 50% by weight of a 2-substituted 4,4,7,7-tetramethyl-1,3-dioxacycloheptane of the general formula I as claimed in claim 1.

7. A process for preparing a 2-substituted 4,4,7,7-tetramethyl-1,3-dioxacycloheptane of the general formula I as claimed in claim 1, which comprises reacting an aldehyde of the general formula II

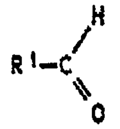

(II)

where $R^1$ is as defined in claim 1, in a conventional manner with 2,5-dimethylhexane-2,5-diol of the formula III

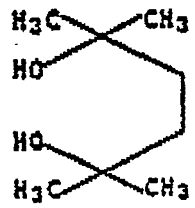

(III)

8. A process for preparing a 4,4,7,7-tetramethyl-1,3-dioxacycloheptane of the general formula I as claimed in claim 1, which comprises hydrogenating the corresponding 4,4,7,7-tetramethyl-1,3-dioxa-5-cycloheptene of the general formula IV

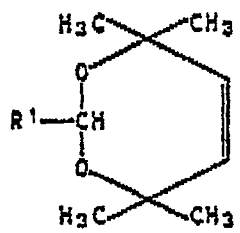

(IV)

in a conventional manner.

**Revendications**

1. 4,4,7,7-tétramethyl-1,3-dioxacycloheptanes substitués en 2, de formule générale

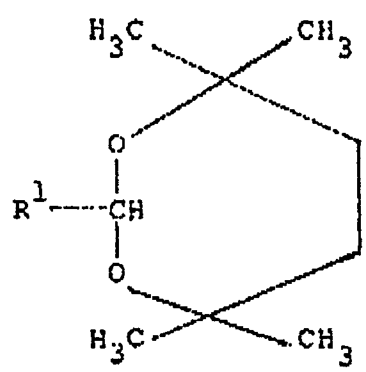

(I)

dans laquelle $R^1$ représente un reste alkyle à chaîne droite ou ramifié de 1 à 10 atomes C, qui peut additionnellement contenir de l'oxygène sous forme d'un groupement ether, ou un noyau cycloalkyle de 5 à 8 chaînons ayant jusqu'à 10 atomes C, qui peut contenir additionnellement de l'oxygène sous forme d'un groupement ether.

2. 2,4,4,7,7-pentamethyl-1,3-dioxacycloheptane.

3. 2-tert.-butyl-4,4,7,7-tetramethyl-1,3-dioxacycloheptane.

4. 2-isopropyl-4,4,7,7-tétramethyl-1,3-dioxacycloheptane.

5. Utilisation des 4,4,7,7-tétramethyl-1,3-dioxacycloheptanes substitués en 2, de formule I selon la revendication 1 comme produits odorants.

6. Compositions odorantes, caractérisées par une teneur de 1 à 50% en poids en 4,4,7,7-tétraméthyl-1,3-diocycloheptanes substituées en 2 de formule générale I selon la revendication 1.

7. Procédé de préparation de 4,4,7,7-tétramethyl-1,3-dioxacycloheptanes substituées en 2 de formule générale I selon la revendication 1, caractérisé par le fait qu'on fait réagir de manière connue en soi un

aldéhyde de formule générale II

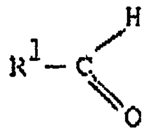

(II)

dans laquelle $R^1$ a la signification indiquée dans la revendication 1 avec un 2,5-diméthyl-hexane-2,5-diol de formule III.

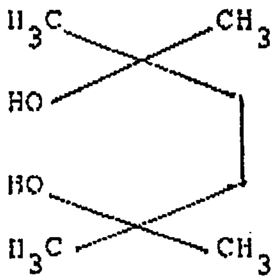

(III)

8. Procédé de préparation de 4,4,7,7-tétraméthyl-1,3-dioxacycloheptanes de formule générale I selon la revendication 1, caractérisé par le fait qu'on hydrogène, de manière connue en soi, les 4,4,7,7-tétra-methyl-1,3-dioxa-5-cycloheptènes de formule générale IV

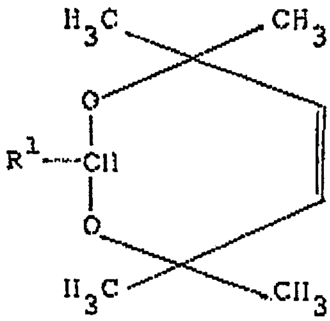

(IV)